# EUROPEAN PATENT SPECIFICATION

(11) **EP 2 481 554 B1**
(45) Date of publication and mention of the grant of the patent: **16.12.2015**
(21) Application number: 11186747.9
(22) Date of filing: 26.10.2011
(51) Int. Cl.: B29C 67/00, A61F 2/00, A61L 27/10, A61L 27/14, B29C 33/38, B29C 39/00, B29C 69/02, A61L 27/56, B29L 31/00

(54) **Method for producing bone substitutes and/or fillers made to measure and made of bioactive and biomimetic materials**
Verfahren zur Massanfertigung eines Knochensubstituts oder eines Knochenfüllers aus biologisch wirksamen und biomimetischen Materialen
Méthode de production sur mesure d'un substitut osseux ou d'un comblement osseux dans des matériaux bioactifs et biomimétiques

(30) Priority: 27.01.2011 IT MI20110094
(43) Date of publication of application: 01.08.2012
(73) Proprietor: Ghimas S.P.A., 40033 Casalecchio di Reno (BO) (IT)
(72) Inventor: Gervaso, Francesca, 73100 LECCE (IT); Scalera, Francesca, 72023 MESAGNE BR (IT); Licciulli, Antonio Alessandro, 72023 MESAGNE BR (IT); Sannino, Alessandro, 73100 LECCE (IT)
(74) Representative: Modiano, Micaela Nadia

(56) References cited:
- EP-A1- 1 584 308
- WO-A1-03/000480
- US-A- 5 641 323
- US-A- 5 897 592

## Description

The present invention relates to a method for producing bone substitutes and/or fillers made to measure and made of bioactive and biomimetic materials.

Bone defects are very common in the population and do not only affect the elderly but can also affect both adults and young people.

The main reason for this spread lies in the fact that the lack of more or less extensive bone portions can arise from a multitude of causes which lead to the necessity to provide bone substitutes or fillers.

For example, some of these causes are tumors, traumas and road accidents, bacterial and viral infections, and dental implantation.

Although technological progress has enabled the development of increasingly effective and efficient methods and procedures, presently orthopedic and maxillofacial surgeons still do not have a standardized process to which to refer in order to obtain a good result both functionally and aesthetically in the reconstruction of a portion of bone of varying size.

Currently, in fact, the most widely adopted solution for the restoration of a bone defect is the use of autologous substitutes which however suffer a number of major drawbacks linked, for example, to the state of health of the donor (if a transplant of tissue occurs from one person to another), the vascularization time and the difficulty of the surgical technique.

In maxillofacial surgery, another major drawback of the above solution consists in the difficulty of obtaining a structure that is symmetric or more generally aesthetically presentable in the attempt to produce a bone substitute that perfectly fits the bone defect to be filled, and in the difficulty of obtaining it with a material that is as similar as possible to human bone.

In order to overcome the above mentioned drawbacks, thanks to the development of reverse engineering techniques applied to biomedical imaging, it is possible to carry out the virtual reconstruction of anatomical portions through the use of software that processes two-dimensional clinical images generated by CAT (computerized axial tomography), NMR (nuclear magnetic resonance), or XR techniques (X-rays).

With the technologies available today, a made-to-measure bone substitute can be obtained by way of a first "direct" method, i.e. by way of photopolymerization of the bone defect in a suitable material using a machine for rapid prototyping (stereolithography, STL) which is capable of reading information about the shape and dimensions of the defect from a suitably formatted file; or by way of a second "indirect" method, i.e. by way of photopolymerization of a mold of the bone defect (again obtained with STL techniques) in which to form the substitute in a preselected material.

More specifically, in the "direct" method first of all the clinical images obtained through standard diagnostic examinations, such as for example CAT or NMR, are processed and then converted to a format supported by the machine for rapid prototyping. Then, once the virtual model of the bone defect to be made is obtained and the most suitable material for the type of application selected, the made-to-measure prototype can easily be produced.

This "direct" method however is not devoid of drawbacks, among which is the fact that the bone substitute which can be made by it cannot be said to be bioactive or biomimetic in any way.

This drawback is inherent in the direct method and concerns both the material selected to make the bone substitute and also the microstructure that the substitute is provided with.

In fact, the materials that are best appropriate to be photopolymerized are polymers based on acrylic, vinyl or epoxy resins, which however are not generally biocompatible and are chemically and structurally very different from the components of the natural tissue that they will replace.

Alternatively, other materials can be selected which are more biomimetic and bioactive, like natural polymers or ceramic materials, which however require chemical modifications in order to be photopolymerized and which, in any case, do not allow any control over the microstructure and over the porosity inside the substitute.

WO 03/000480 A1 discloses a method for producing bone substitutes or fillers made to measure.

The aim of the present invention consists in devising a method for producing bone substitutes and/or fillers made to measure in such a manner as to perfectly fit the anatomical portion to be substituted and which offer morphological, physical and mechanical characteristics that are designed to reproduce as faithfully as possible the morphological, physical and mechanical characteristics of the natural tissues that they will replace.

Within this aim, an object of the present invention is to devise a method for producing bone substitutes and/or fillers made to measure that are made of a material that is bioactive and biomimetic simply and rapidly, by availing of the technologies currently present on the market.

This aim and these and other objects which will become better apparent hereinafter are achieved by a method for producing bone substitutes and/or fillers made to measure and made of bioactive and biomimetic materials according to claim 1.

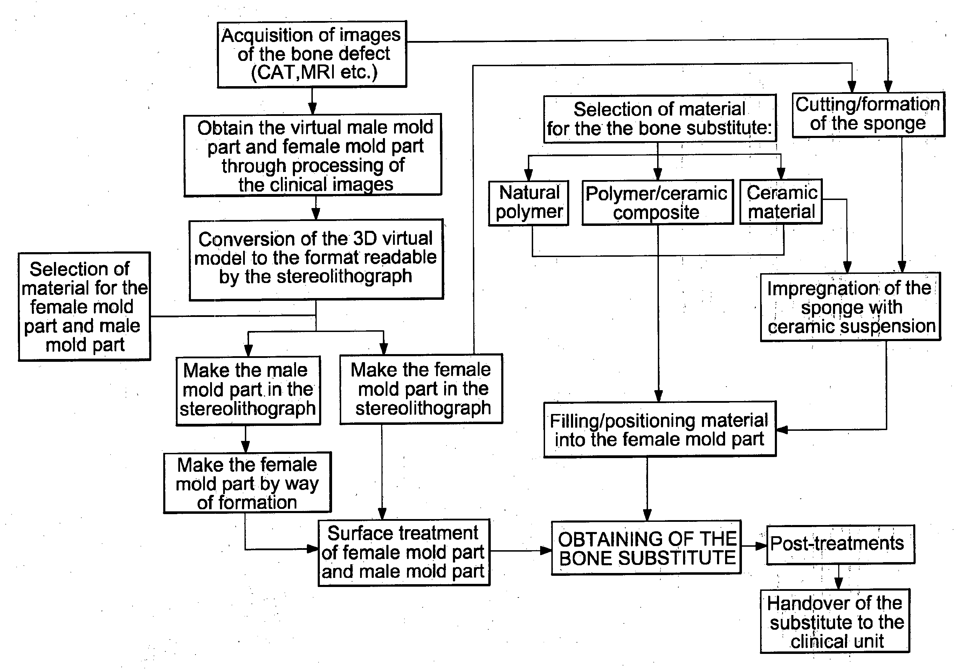

Further characteristics and advantages of the invention will become better apparent from the detailed description of a preferred, but not exclusive, embodiment of a method for producing bone substitutes and/or fillers made to measure and made of bioactive and biomimetic materials, illustrated by way of non-limiting example in the accompanying drawing wherein a block diagram showing the method is represented.

With reference to the figure, the method for producing bone substitutes and/or fillers made to measure and made of bioactive and biomimetic materials, according to the invention, comprises a first sequence of steps comprising:
- receiving clinical images of the bone defect from a clinical unit,
- processing the received image in order to obtain the virtual prototype of the female mold part and male mold part of the bone defect,
- converting the virtual prototype into a format which is compatible with a rapid prototyping machine.

More specifically, the acquisition of clinical images of the anatomical portion to be reconstructed (defect) can occur using the instrumentation available to the clinical units (hospitals, analysis laboratories, dental studios etc.) such as for example CAT, NMR, or XR.

Nonetheless, in the step of processing the image it is also possible to correctly handle, in addition to the images sourced from routine instrumentation, images acquired with cone beam CT, i.e. with a computerized tomography that is considerably less invasive than the traditional kind.

In fact, the processing of the clinical images of the anatomical portion to be substituted occurs using adapted software to obtain the three-dimensional virtual model of the defect and of the negative of the defect.

In this manner it is possible to obtain a virtual model of the defect which is used to make a male mold part made of resin by way of stereolithography. For example, from this resin male mold part it is possible to obtain a female mold part made of silicone for the easy extraction of a bone substitute having a complex shape, or made of ceramic material and thus capable of withstanding high sintering temperatures, or made of metallic material so as to have high thermal or electrical conductivity. In another embodiment, which is not part of the invention, the virtual model of the negative of the defect is used to create the resin female mold part by way of stereolithography.

Conveniently, in the processing step, the images are processed in such a manner as to obtain an increase in the dimensions of the female mold part and of the male mold part in order to take into account any shrinkage of material as a consequence of the processes used in the step of making the bone filler or substitute, which will be better described hereinafter.

Once the images are processed and the virtual model of the defect obtained and the necessary conversions of the information contained in the virtual model performed, a second sequence of steps is performed which comprise:
- selecting the material of which the female mold part and the male mold part are to be made, in order to provide female mold parts and male mold parts made of different materials depending on the type of production method used to obtain the bone substitute,
- providing the male mold part by way of the rapid prototyping machine.

More precisely, the selection of the material with which to make the female mold part and the male mold part is preferably comprised among the following materials: synthetic polymers based on acrylic, vinyl or epoxy resins, natural polymers (such as, for example, collagen and protein in general, cellulose and other polysaccharides) which need to be modified in order to be photopolymerized by way of stereolithography, ceramic materials, composites of the preceding materials.

Once the material with which to make the female mold part and the male mold part is selected, the creation of the male mold part by way of the stereolithography technique is performed.

In more detail, for example, a female mold part made of ceramic material is obtainable by way of forming a ceramic suspension in a male mold part made of resin, provided by way of rapid prototyping or alternatively according to an embodiment which does not form part of the invention, directly by way of rapid prototyping using a ceramic material that has previously been functionalized or conveniently mixed with a polymer so as to render it susceptible to photopolymerization.

In the same manner, it is possible to obtain the female mold part by way of forming silicone in a male mold part made of resin, in order to facilitate the extraction of bone substitutes having a complex shape.

Moreover, it is possible to obtain the female mold part and the male mold part by way of rapid prototyping in a metallic material with high thermal and electrical conductivity according to an embodiment which does not form part of the invention.

Advantageously, if deemed necessary, a step of surface treatment can be provided of the inner surfaces of the female mold part and of the outer surfaces of the male mold part in order to obtain a preset surface roughness which facilitates biological integration between the bone filler or substitute and the seat of the implant.

With the female mold part and the male mold part defined, the steps involving the obtaining of the bone filler or substitute are performed.

First of all, the material is selected with which to make the bone filler or substitute. This step is not to be considered independent of the others; in fact, the selection of material with which to make the bone filler or substitute is closely linked to the selection of material with which the female mold part and the male mold part are made, as well as the method of making the bone filler or substitute proper.

More specifically, the bone filler or substitute is made preferably of a material selected among the following materials: natural polymers (such as, for example, collagen and proteins in general, cellulose and other polysaccharides), ceramic materials, composites of the preceding materials.

If it is decided to obtain a bone filler or substitute made of ceramic material, then a sponge is obtained of the appropriate shape and dimensions. In order to obtain a bone substitute made of a ceramic material with high and controlled porosity it is possible to use the polymeric sponge replication method. This technique involves the impregnation of a selected sponge with a ceramic suspension, the elimination of the sponge at temperature and the subsequent compaction of the ceramic powders at high temperatures (sintering). In this case it is necessary that the sponge in question be conveniently contoured in the shape and size of the defect so that it perfectly fits once the sponge is impregnated.

The sponge can then be cut with a laser using as input the three-dimensional virtual model of the defect generated previously or by providing the sponge directly inside the female mold part of the defect thus obtaining the sponge with a geometry that is exact.

In this manner it is also possible to design a sponge with a porosity that is modulated so as to obtain a corresponding porosity.

The bone substitute is then prepared through the impregnation of the conveniently contoured sponge with a ceramic suspension or through the pouring of the selected material into the female mold part.

Advantageously, the step of making the bone filler or substitute is performed with one of the following methods depending on the material selected for the bone filler or substitute: freeze-drying, burn-out and sintering, drying.

More precisely, the burn-out and sintering technique is a thermal treatment process, with or without application of outer pressure, by way of which a system of individual particles or a porous body modifies its properties thus evolving towards a state of maximum density and minimum porosity.

During the sintering, phase transformations and chemical reactions occur at the same time, and the microstructural and macrostructural formation of the end product takes place.

After the sintering it is difficult to intervene in order to change the shape and structure of ceramics.

Porosity is thus the fundamental parameter for describing the process of sintering of ceramic materials.

In particular, in the first step of the firing the mixing water is eliminated (at around 100°C), as well as the structural water and the organic bonds (at 400-500°C, burn-out step). In the next step of sintering the product passes from a highly porous one to a compact one. From a thermodynamic viewpoint, the driving force that causes the conversion of compacted powders (green ceramic) to a dense solid is the reduction of total free energy. In traditional sintering the reduction of such superficial energy is achieved by way of aggregation of multiple small particles into fewer larger ones, and by way of the replacement of gas/solid interfaces with other, solid/solid interfaces.

With regard to the technique of freeze-drying, which is nothing more than a method of lyophilization, this is a technological process that makes it possible to eliminate the water from an organic matrix with the minimum possible deterioration of the structure and of the components of the matrix proper.

This method basically comprises two physical processes: freezing and sublimation. The principle of the method involves the application of heat to the frozen sample kept in a vacuum. The water contained in the product in the form of ice is extracted directly as vapor by sublimation, since the working pressure is much lower than the triple point of the water, i.e. at conditions that allow the simultaneous presence of water in the three phases, solid, liquid, vapor.

The water vapor extracted is captured for freezing on cooling coils called condensers; the non-condensable gases are aspirated and removed by the vacuum pump. The process is carried out under carefully controlled temperature and pressure conditions in order to prevent damage to the structure of the product, so that the original matrix is almost perfectly recoverable when, at the time of use, it is desired to proceed with rehydration.

Finally, a step of post-treatment can be provided of the bone filler or substitute, by way of covering it with a thin surface coating adapted to increase its osteointegration and/or filling it with a material adapted to increase its biomimicry and its bioactivity.

More precisely, if the bone substitute has been made of collagen with the freeze-drying technique it can, for example, be subjected to a thermal reticulation process in order to increase its mechanical strength.

Other examples of post-treatment are constituted by the infiltration of the ceramic sponge described previously with a suspension of collagen and a subsequent cycle of lyophilization in order to obtain a ceramic/collagen composite that is highly biomimetic; or, as mentioned previously, by coatings of the surface of the substitute with bioactive materials adapted to favor the integration of the substitute within the organism.

At any rate, once the bone substitute is handed over to the clinical unit, the latter can implant it in the patient by way of surgical intervention.

In practice it has been found that the method for producing bone substitutes and/or fillers made to measure and made of bioactive and biomimetic materials , according to the present invention, achieves the intended aim and objects in that they make it possible to obtain the defect of the desired shape as in the conventional direct method but made of materials that exhaustively mimic the nature of the biological tissues to be reintegrated, exhibiting the necessary porosities for cellular recolonization.

Another advantage of the production method, according to the present invention, consists in obtaining substitutes made of natural polymers with porosity of which both size and orientation are controlled using the directional freeze-drying technique, which obtains patterns of conveniently designed ice crystals which will result in corresponding patterns of porosity.

The invention, thus conceived, is susceptible of numerous modifications and variations, all of which are within the scope of the appended claims.

Moreover, all the details may be substituted by other, technically equivalent elements.

In practice the materials employed, provided they are compatible with the specific use, and the contingent dimensions and shapes, may be any according to requirements.

## Claims

1. A method for producing bone substitutes and/or fillers made to measure and made of bioactive and biomimetic materials, **characterized in that** it comprises the following steps:
- receiving clinical images of the bone defect from a clinical unit,
- processing the received image in order to obtain a virtual prototype of the bone defect,
- converting the virtual prototype into a format which is compatible with a rapid prototyping machine,
- providing a male mold part by way of said rapid prototyping machine, said machine being a storeolithography machine, from said virtual prototype of said bone defect, in a material selected depending on the type of production method used to obtain the bone substitute,
- obtaining by forming from said male mold part a female mold part made of silicone, or ceramic suspension depending on the type of production method used to obtain the bone substitute,
- selecting a material of which the bone filler or substitute is to be made,
- providing said bone filler or substitute.

2. The production method according to claim 1, **characterized in that** said bone filler or substitute is made of a material selected among the following materials: natural polymers, ceramic materials, composites of the preceding materials.

3. The production method according to one or more of the preceding claims, **characterized in that** said step of producing said bone filler or substitute is performed with one of the following methods, depending on the material selected for said bone filler or substitute: freeze-drying, burn-out and sintering, drying.

4. The production method according to one or more of the preceding claims, **characterized in that** it comprises a step of surface treatment of said female mold part and male mold part in order to obtain a preset surface roughness which facilitates biological integration between said bone filler or substitute and the seat of the implant.

5. The production method according to one or more of the preceding claims, **characterized in that** it comprises a step of post-treatment of said bone filler or substitute by covering it with a surface coating adapted to increase its osteointegration and/or by filling it with a material adapted to increase its biomimicry and its bioactivity.

6. The production method according to one or more of the preceding claims, **characterized in that** in said processing step the images are processed in such a manner as to obtain an increase in the dimensions of said female mold part and male mold part in order to take into account any shrinkage of material as a consequence of the processes used during the step of production of said bone filler or substitute.

7. The production method according to one or more of the preceding claims, **characterized in that** it comprises a step of obtaining a sponge having the desired shape and dimensions by obtaining said bone filler or substitute made of a ceramic material with high and controlled porosity.

8. The production method according to one or more of the preceding claims, **characterized in that** said female mold part is obtained by forming silicone in a male mold part made of resin in order to facilitate the extraction of bone substitutes having a complex shape.

## Patentansprüche

1. Ein Verfahren zur Herstellung von Knochenersatzmitteln und/oder -füllungen, maßgeschneidert und hergestellt aus bioaktiven und biomimetischen Materialien, **dadurch gekennzeichnet, dass** es folgende Schritte umfasst:
- Empfangen klinischer Bilder des Knochendefekts von einer klinischen Einheit,
- Verarbeitung des empfangenen Bildes, um einen virtuellen Prototyp des Knochendefekts zu erhalten,
- Umwandlung des virtuellen Prototyps in ein Format, das mit einer Maschine für schnelle Prototypentwicklung kompatibel ist,
- Bereitstellung eines Patrizenteils mit Hilfe der Maschine für schnelle Prototypentwicklung, wobei die Maschine eine Stereolithographie-Maschine ist, aus dem virtuellen Prototypen des Knochendefekts, aus einem Material, das gewählt ist in Abhängigkeit von der Art des Produktionsverfahrens, das angewandt wird, um das Knochenersatzmittel herzustellen,
- Gewinnung, durch Formen aus dem Patrizenteil eines Matrizenteils aus Silikon oder einer Keramiksuspension, in Abhängigkeit von der Art des Produktionsverfahrens, das zur Herstellung des Knochenersatzmittels angewandt wird,
- Auswahl eines Materials, aus dem die Knochenfüllung oder das Knochenersatzmittel hergestellt werden soll,
- Bereitstellung der Knochenfüllung oder des Knochenersatzmittels.

2. Das Herstellungsverfahren gemäß Anspruch 1, **dadurch gekennzeichnet, dass** die Knochenfüllung oder das Knochenersatzmittel aus einem Material besteht, das gewählt ist aus den folgenden Materialien: natürlichen Polymeren, Keramikmaterialien, Verbundstoffen der oben genannten Materialien.

3. Das Herstellungsverfahren gemäß einem oder mehreren der obigen Ansprüche, **dadurch gekennzeichnet, dass** der Schritt der Herstellung der Knochenfüllung oder des Knochenersatzmittels mit einem der folgenden Verfahren durchgeführt wird, je nach dem Material, das für die Knochenfüllung oder das Knochenersatzmittel gewählt wird: Gefriertrocknung, Ausbrennen und Sintern, Trocknung.

4. Das Herstellungsverfahren gemäß einem oder mehreren der obigen Ansprüche, **dadurch gekennzeichnet, dass** es einen Schritt der Oberflächenbehandlung des Matrizenteils und des Patrizenteils, um eine vordefinierte Oberflächenrauheit zu erhalten, die die biologische Integration zwischen der Knochenfüllung oder dem Knochenersatzmittel und dem Sitz des Implantats erleichtert, umfasst.

5. Das Herstellungsverfahren gemäß einem oder mehreren der obigen Ansprüche, **dadurch gekennzeichnet, dass** es einen Schritt der Nachbehandlung der Knochenfüllung oder des Knochenersatzmittels durch Bedeckung derselben/desselben mit einer Oberflächenbeschichtung umfasst, die geeignet ist, ihre/seine Osteointegration zu erhöhen, und/oder durch Füllen derselben/desselben mit einem Material, das geeignet ist, ihre/seine Biomimikry und Bioaktivität zu erhöhen.

6. Das Herstellungsverfahren gemäß einem oder mehreren der obigen Ansprüche, **dadurch gekennzeichnet, dass** in dem Verarbeitungsschritt die Bilder so verarbeitet werden, dass eine Vergrößerung der Maße des Matrizenteils und des Patrizenteils erzielt wird, zum Zwecke der Berücksichtigung einer eventuellen Schrumpfung von Material infolge der Verfahren, die während des Schritts der Herstellung der Knochenfüllung oder des Knochenersatzmittels angewandt werden.

7. Das Herstellungsverfahren gemäß einem oder mehreren der obigen Ansprüche, **dadurch gekennzeichnet, dass** es einen Schritt der Herstellung eines Schwamms umfasst, der die gewünschte Form und die gewünschten Maße hat, durch Herstellung der Knochenfüllung oder des Knochenersatzmittels aus einem Keramikmaterial mit hoher und kontrollierter Porosität.

8. Das Herstellungsverfahren gemäß einem oder mehreren der obigen Ansprüche, **dadurch gekennzeichnet, dass** das Matrizenteil hergestellt wird durch Formen von Silikon in einem Patrizenteil, das aus Harz besteht, um die Extraktion von Knochenersatzmitteln mit komplexer Form zu erleichtern.

## Revendications

1. Méthode pour produire des substituts d'os et/ou des charges osseuses réalisés pour mesurer et constitués de matériaux bioactifs et biomimétiques, **caractérisée en ce qu'**elle comprend les étapes suivantes :
- de réception d'images cliniques du défaut osseux d'une unité clinique,
- de traitement de l'image reçue afin d'obtenir un prototype virtuel du défaut osseux,
- de conversion du prototype virtuel en un format qui est compatible avec une machine de prototypage rapide,
- de réalisation d'une partie de moule mâle au moyen de ladite machine de prototypage rapide, ladite machine étant une machine de stéréolithographie, à partir dudit prototype virtuel dudit défaut osseux, en un matériau sélectionné en fonction du type de méthode de production utilisée pour obtenir le substitut d'os,
- d'obtention, par formation à partir de ladite partie de moule mâle, d'une partie de moule femelle constituée de silicone, ou d'une suspension de céramique en fonction du type de méthode de production utilisée pour obtenir le substitut d'os,
- de sélection d'un matériau en lequel la charge osseuse ou le substitut d'os doit être réalisé,
- de fourniture de ladite charge osseuse ou dudit substitut d'os.

2. Méthode de production selon la revendication 1, **caractérisée en ce que** ladite charge osseuse ou ledit substitut d'os est réalisé en un matériau sélectionné parmi les matériaux suivants : des polymères naturels, des céramiques, des composites des matériaux précédents.

3. Méthode de production selon une ou plusieurs des revendications précédentes, **caractérisée en ce que** ladite étape de production de ladite charge osseuse ou dudit substitut d'os est effectuée par l'une des méthodes suivantes, en fonction du matériau sélectionné pour ladite charge osseuse ou ledit substitut d'os : une lyophilisation, une caléfaction et un frittage, un séchage.

4. Méthode de production selon une ou plusieurs des revendications précédentes, **caractérisée en ce qu'**elle comprend une étape de traitement de surface de ladite partie de moule femelle et de ladite partie de moule mâle afin d'obtenir une rugosité de surface présélectionnée qui facilite une intégration biologique entre ladite charge osseuse ou ledit substitut d'os et le siège de l'implant.

5. Méthode de production selon une ou plusieurs des revendications précédentes, **caractérisée en ce qu'**elle comprend une étape de post-traitement de ladite charge osseuse ou dudit substitut d'os en le recouvrant avec un revêtement de surface adapté pour augmenter son ostéointégration et/ou en le remplissant avec un matériau adapté pour augmenter son biomimétisme et sa bioactivité.

6. Méthode de production selon une ou plusieurs des revendications précédentes, **caractérisée en ce que**, à ladite étape de traitement, les images sont traitées de manière à obtenir une augmentation des dimensions de ladite partie de moule femelle et de ladite partie de moule mâle afin de prendre en compte tout retrait de matériau dû aux processus utilisés pendant l'étape de production de ladite charge osseuse ou dudit substitut d'os.

7. Méthode de production selon une ou plusieurs des revendications précédentes, **caractérisée en ce qu'**elle comprend une étape d'obtention d'une éponge ayant la forme et les dimensions souhaitées en obtenant ladite charge osseuse ou ledit substitut d'os réalisé en céramique avec une porosité élevée et contrôlée.

8. Méthode de production selon une ou plusieurs des revendications précédentes, **caractérisée en ce que** ladite partie de moule femelle est obtenue par formage de silicone dans une partie de moule mâle réalisée en résine afin de faciliter l'extraction de substituts d'os ayant une forme complexe.
